# EUROPÄISCHE PATENTSCHRIFT

(11) **EP 2 230 965 B1**
(45) Veröffentlichungstag und Bekanntmachung des Hinweises auf die Patenterteilung: **28.09.2016**
(21) Anmeldenummer: 09700688.6
(22) Anmeldetag: 02.01.2009
(51) Int. Cl.: A46B 5/04

(54) **VORRICHTUNG ZUR REINIGUNG DES MUNDRAUMS**
DEVICE FOR CLEANING THE ORAL CAVITY
DISPOSITIF DE NETTOYAGE DE LA CAVITÉ BUCCALE

(30) Priorität: 10.01.2008 DE 202008000384 U
(43) Veröffentlichungstag der Anmeldung: 29.09.2010
(73) Patentinhaber: Hilpert, Norbert, 87669 Rieden (DE)
(72) Erfinder: Methfessel, Thomas, 87480 Weitnau-Hofen (DE)
(74) Vertreter: Hentrich Patentanwälte PartG mbB
(86) Internationale Anmeldenummer: PCT/DE2009/000001
(87) Internationale Veröffentlichungsnummer: WO 2009/086813

(56) Entgegenhaltungen:
- WO-A-96/16610
- WO-A1-03/096923
- WO-A2-01/76523
- DE-C1- 10 215 805
- GB-A- 2 099 305
- JP-A- 2005 253 872
- US-A- 5 440 774
- US-A- 5 953 783

## Beschreibung

Die vorliegende Erfindung betrifft eine Vorrichtung zur Reinigung des Mundraums, welche eine Grundform eines Fingerhuts mit der Eignung des Ziehens über einen Finger aufweist und die Grundform mit einer Sicherung versehen ist.

Derartige Vorrichtungen zur Reinigung des Mundraums sind bereits in vielfältiger Form und Ausgestaltung bekannt und gebräuchlich. Die Reinigung des Mundraums, insbesondere der Zähne und des Zahnfleisches, spielt in der Gegenwart eine immer wichtigere Rolle. Dabei werden an die genutzten Vorrichtungen immer höhere Ansprüche an Effizienz und Hygiene gestellt.

So ist beispielsweise aus der DE 102 15 805 C1 ein sog. Zahnreinigungsteil bekannt, das mit einer gewirkten oder gestrickten Grundschicht, welche die Form einer am einen Ende geschlossenen Stulpe aufweist, und mit einer von der Grundschicht getragenen Florschicht, welche von der Grundschicht nach Außen überstehende Fäden aufweist, wobei die Florschicht eine mit der Grundschicht mitgewirkte oder mitgestrickte Schicht aus Schlingen umfasst.

Die US 5 953 783 A offenbart eine Zahnreinigungsvorrichtung, welche unter anderem eine Grundform eines Fingerhuts aufweist, wobei eine elastische Klemmeinrichtung vorgesehen ist, um ein Abrutschen der Vorrichtung von dem Finger zu verhindern.

Die GB 2 099 305 A beschreibt ebenfalls eine Vorrichtung zum Reinigen insbesondere von Zähnen, welche aus einem gewebten oder nicht gewebten Faserfingerling besteht. Um ein Abrutschen des Fingerlings vom Finger zu verhindern ist der Fingerling mit einer elastischen Kante versehen oder einer anderen passenden Einfassung.

Die US 5 440 774 A bezieht sich auf einen Einwegartikel zum Reinigen von Zähnen. Dieser wird ebenfalls über einen Finger gezogen. Als Sicherung gegen ein Abrutschen der Vorrichtung vom Finger sind Kanten vorgesehen, welche zwischen dem Daumen und dem Finger des Benutzers eingeklemmt werden und so das Abrutschen verhindern sollen. Ein weiteres Ausführungsbeispiel zeigt Erhöhungen, welche, in der Regel in Zusammenwirken mit dem Daumen des Benutzers, ebenfalls ein Abrutschen verhindern sollen.

Aus der WO 96/16610 A geht ebenfalls eine Art Fingerhut zur Reinigung von Zähnen hervor. Eine Sicherung gegen ein Abrutschen ds Fingers vom Finger ist nicht vorgesehen.

Nachteilig an dem Stand der Technik ist, dass sich der Nutzer mit zu großen Fingern oder zu kleinen Fingern oftmals schwer tut, da eine Vorrichtung nach dem Stand der Technik entweder zu lasch oder zu fest auf seinem Finger sitzt und in beiden Fällen droht, vom Finger zu rutschen. Außerdem bildet der feingewobene Stoff einen guten Hort für Bakterien oder dergleichen, welche bei späteren Benutzungen wieder auf das Zahnfleisch oder womöglich vorhandene Zahnfleischverletzungen aufgebracht werden.

Darüber hinaus ist die WO 03/096923A1 bekannt, in der eine Vorrichtung zur Reinigung des Mundraums beschrieben wird, welche eine Grundform eines Fingerhuts aufweist, die über einen Finger gezogen werden kann, sowie eine mit der Grundform verbundene als Schlaufe gebildete Sicherung. Die Vorrichtung ist aus Latex gebildet, wobei an der Grundform Borsten zum Reinigen des Mundraums angebracht sind. Nachteilig wirkt sich zum einen aus, dass es sich um einen Einwegartikel handelt und zum anderen können die Borsten zu Verletzungen im Mundraum speziell am Zahnfleisch und an den Zahnhälsen führen.

Der vorliegenden Erfindung liegt die Aufgabe zugrunde, eine Vorrichtung zur Reinigung des Mundraums zu schaffen, welche einfach und effektiv in der Handhabung ist und dabei auch bei mehrmaliger Benutzung hygienisch bleibt.

Die erfindungsgemäße Aufgabe wird bei einer Vorrichtung der eingangs genannten Art durch den kennzeichnenden Teil des Anspruchs 1 gelöst.

Eine erfindungsgemäße Vorrichtung zur Reinigung des Mundraums kann aus verschiedenen Materialien hergestellt werden. Im vorliegenden Ausführungsbeispiel handelt es sich bei den verwandten Materialien um Mikrofasergewebe.

Mikrofaser ist hierbei eine Bezeichnung für Fasern, deren Einzelfäden aus einer Polyester-Polyamid-Mischung hergestellt werden und feiner als 1 Denier sind. Feine Seide zum Beispiel hat ca. 1,24 Denier. Die meisten Mikrofasern liegen bei 0,5 bis 0,6 Denier. Der einzelne Faden des genutzten Fasergewebes hat nur einhundertstel des Durchmessers eines menschlichen Haares. Wie die Fasern so klein sind, können viele von ihnen zusammengepackt werden, was zu einer größeren Fadenoberfläche führt Mikrofasergewebe sind außergewöhnlich weich und halten sehr gut ihre Form. Ein entscheidender Vorteil aller Mikrofasertextilien ist ihre besonders starke Saugfähigkeit. Sie nehmen fünf Mal so viel Wasser auf, wie herkömmliche Baumwollfasern. Dies ist eine wichtige Eigenschaft wenn es um die Anwendung im oralen Bereich geht. Dort ist aufgrund des vom Nutzer eingebrachten Wassers in den Mund zur Reinigung sowie des Speichels ein sehr feuchtes Klima.

Die antimikrobielle Wirksamkeit der genutzten Textilien basiert auf Silberionen, die fest im Faserpolymer eingebunden sind. Auf bioaktiven Textilien wird das Wachstum von Bakterien gehemmt. Die antimikrobielle Wirkung erfolgt nur unmittelbar auf dem Textil und wird nicht freigesetzt. Somit ist die Verwendung des Textils sicher für den Verbraucher.

Die genutzten Materialien sind hier nicht als abschliessend zu betrachten. Vielmehr ist es von Bedeutung, ob die genutzten Materialien die an sie gestellten Aufgaben erfüllen können. Ist dies der Fall, so kommen sie als Materialien für die Vorrichtung zur Reinigung des Mundraums in Betracht.

Die Aufgaben sollen hier nicht abschliessend aber beispielhaft aufgezählt werden. Zunächst ist darauf abzustellen, dass eine Grundschicht aus einem flexiblen Material gestaltet sein muss, das sich leicht weben, stricken od. dgl. verarbeiten lässt.

Auf der Grundschicht wird eine Florschicht aufgebracht. Bei dieser Florschicht ist auf andere Eigenschaften Wert zu legen. Denn an sie werden in erster Linie folgende Aufgaben gestellt. Neben der Aufnahme von Flüssigkeiten, v. a. eine antimikrobielle Wirkung zu entfalten und sich nach der Anwendung leicht reinigen zu lassen.

Eine weitere Möglichkeit der kostengünstigen Herstellung ist in folgender Weise ebenfalls vom Erfindungsgedanken mit umfasst. Dabei werden zwei Mikrofasertücher aufeinander gelegt und durch ein U-Pofil-Stanzmesser auf die gewünschte Form und Grösse gestanzt. Zugleich werden mit Ultraschall-Schweisstechnik die Stanzräder miteinander verbunden. Die Sicherheitsschleife kann in diesem Arbeitsschritt auch gleich mit angebracht werden. Der nun fertige Fingerling wird nun noch von links auf rechts umgedreht.

Eine weitere wichtige Aufgabe der Florschicht ist, dass sie sich leicht mit der Grundschicht verbinden lassen muss. Im bevorzugten Ausführungsbeispiel ist an die Grundform die Sicherung angebracht.

Die Art der Anbringung der Sicherung ist nicht von Belang. Ebenso ist es nicht wichtig, dass die Sicherung aus demselben Material besteht wie die Grundschicht. Dies erleichtert nur die Verarbeitung und gewährleistet die Flexibilität und Dehnbarkeit der Sicherung.

Die Sicherung ist im bevorzugten Ausführungsbeispiel als Schlaufe ausgebildet und dient dem Überstülpen auf einen Mittelfinger. Vom Erfindungsgedanken abgedeckt sollen ebenfalls andere Formen der Sicherung der Grundform sein.

So ist es genauso denkbar, das zwei Fäden von der Grundform abstehen, welche der Nutzer um sein Handgelenk wickelt oder dort verknotet, um ein Abgleiten der Vorrichtung bei der Reinigung des Mundraums zu verhindern. Der Erfindungsgedanke geht grundsätzlich dahin, die Vorrichtung vor einem Abgleiten vom Finger zu schützen.

Ausserdem ist die im bevorzugten Ausführungsbeispiel gezeigte Vorrichtung E darauf ausgelegt, dass sie am Zeige- und Mittelfinger der rechten Hand zum Einsatz kommen soll. Unabhängig von dem Ausführungsbeispiel ist vom Erfindungsgedanken ebenfalls die Anordnung an jedem anderen Finger umfasst. So ist es nicht wichtig, auf welchen Finger die Grundform gestülpt wird. Ebenso ist es auch nicht wichtig, welche Hand zur Anwendung der Vorrichtung genutzt wird.

Grundsätzlich ist auch die Ausgestaltung als Handschuh denkbar, wobei neben der Grundform, die in einen oder mehrere Finger integriert sein kann, vom Rest des Handschuhs gesichert wird.

Durch die Sicherung der Grundform ist es einfacher möglich, genügend Kraft auf das Zahnfleisch auszuüben, um es auch zu massieren.

Neben der Nutzung beim Menschen ist auch die Nutzung bei Tieren vom Erfindungsgedanken mit umfasst.

Mundraum bezeichnet neben den Zähnen, Zunge, Zahnfleisch und Mundhöhle auch alle im Mundraum fremden Gegenstände. So ist es z B. denkbar, dass der Nutzer durch eine besonders angepasste Vorrichtung metallene Schmuckstücke, die sich im Mundraum befinden, reinigen oder Zahnfüllungen aus Gold oder Keramik polieren kann.

### Figurenbeschreibung

Weitere Vorteile, Merkmale und Einzelheiten der Erfindung ergeben sich aus der nachfolgenden Beschreibung bevorzugter Ausführungsbeispiele sowie anhand der Zeichnung; Diese zeigt in ihrer einzigen Figur eine erfindungsgemäße Vorrichtung zum Reinigen des Mundraums.

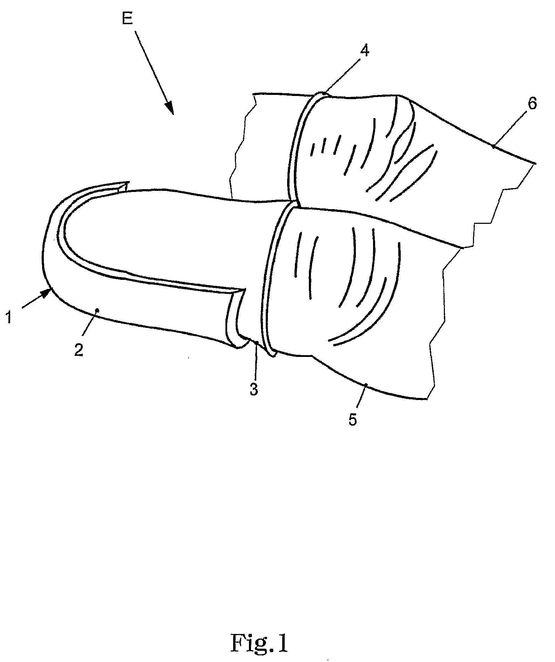

In Fig. 1 ist eine Vorrichtung E zum Reinigen des Mundraums gezeigt. Diese besteht aus einer Grundform 1 und einer Sicherung 4. Sie sind an einer Stelle fest miteinander verbunden.

Die Grundform 1 wiederum besteht aus einer Florschicht 2 und einer Grundschicht 3. Die Grundschicht 3 ist elastisch gestaltet. Sie erstreckt sich unter der Florschicht bis hin zum Ende der Fingerkuppe und umschliesst sie wie ein Fingerhut. Auf der Grundschicht ist die Florschicht im bevorzugten Ausführungsbeispiel bis zur Höhe des Nagelbetts angeordnet. Der Fingerrücken ist nur von der Grundschicht bedeckt.

Die Sicherung 4 ist im bevorzugten Ausführungsbeispiel aus dem gleichen Material gefertigt, wie die Grundschicht 3. Sie ist flexibel und schlaufenartig ausgestaltet.

Die Funktionsweise der vorliegenden Erfindung ist folgende: Der Nutzer zieht sich die Grundform 1 über einen Zeigefinger 5. Im bevorzugten Ausführungsbeispiel handelt es sich dabei um den Zeigefinger 5 der rechten Hand.

Die Sicherung 4 wird über einen Mittelfinger 6 derselben Hand gezogen.

Anschliessend kann der Nutzer die Grundform 1 gesichert durch die Sicherung 4 in den Mundraum einführen und durch hin und her bewegen den Mundraum, insbesondere die Zähne damit reinigen.

### BEZUGSZEICHENLISTE

- 1: Grundform
- 2: Florschicht
- 3: Grundschicht
- 4: Sicherung
- 5: Zeigefinger
- 6: Mittelfinger

- E: Vorrichtung

## Patentansprüche

1. Vorrichtung zur Reinigung des Mundraums, welche eine Grundform (1) eines Fingerhuts mit der Eignung des Ziehens über einen Finger aufweist und die Grundform (1) mit einer Sicherung (4) versehen ist, **dadurch gekennzeichnet, dass** die Grundform (1) aus einer Grundschicht (3) und einer auf die Grundschicht (3) aufgebrachten Florschicht (2) besteht und, aus Mikrofasergewebe mit Fasern feiner als 1 Denier und im Faserpolymer fest eingebundenen Silberionen mit antimikrobieller Wirksamkeit, dass die Sicherung (4) mit der Grundschicht verbunden ist und als eine Schlaufe ausgebildet ist mit der Eignung des Überstülpens auf einen anderen Finger.

2. Vorrichtung nach Anspruch 1, **dadurch gekennzeichnet, dass** die Sicherung (4) flexibel ausgestaltet ist.

## Claims

1. A device for cleaning the oral cavity, having the basic form (1) of a thimble suited to be pulled over a finger, the basic form (1) has a securing means (4), **characterized in that** the basic form (1) consists of a base section (3) and of a pile section (2) fixed on the base section (3), and of microfiber with fibers finer than 1 denier and with silver ions firmly bound in the fiber polymer with antibacterial activity, **in that** the securing means (4) is connected to the base section (3) and designed as a loop suited to be pulled over a different finger.

2. The device as claimed in claim 1 **characterized in that** the securing means (4) is flexible.

## Revendications

1. Dispositif de nettoyage de la cavité buccale ayant une forme de base (1) d'un dé à coudre apte à être disposé sur un doigt, la forme de base (1) étant munie d'un moyen de maintien (4), **caractérisé en ce que** la forme de base (1) est constituée d'une couche de base (3) et d'une couche de voile (2) appliquée sur la couche de base (3), dans un tissu microfibre avec des fibres d'une finesse inférieure à 1 denier et des ions argent qui sont incorporés à demeure dans le polymère des fibres et qui ont une action antimicrobienne, **en ce que** le moyen de maintien (4) est lié à la couche de base et réalisé sous la forme d'une boucle apte à être enfilée sur un autre doigt.

2. Dispositif selon la revendication 1, **caractérisé en ce que** le moyen de maintien (4) est conçu de marnière flexible.
